# EUROPÄISCHE PATENTANMELDUNG

(11) **EP 4 570 782 A1**
(43) Veröffentlichungstag der Anmeldung: **18.06.2025**
(21) Anmeldenummer: 24218078.4
(22) Anmeldetag: 06.12.2024
(51) Int. Cl.: C07C 213/04, C07C 269/02, C07C 271/24, C07C 215/40, C07C 53/126

(54) **TRIMERISIERUNGSKATALYSATOREN**

(30) Priorität: 14.12.2023 EP 23216564
(71) Anmelder: Evonik Operations GmbH, 45128 Essen (DE)
(72) Erfinder: Spyrou, Emmanouil, 46514 Schermbeck (DE); Gollan, Elke, 44653 Herne (DE); Appel, Christine, 45659 Recklinghausen (DE); Klawikowski, Ralf, verstorben (DE); Eggert, Jennifer, 45894 Gelsenkirchen (DE); Kohlstruk, Stephan, 45966 Gladbeck (DE)
(74) Vertreter: Evonik Patent Association

(57) **Zusammenfassung**

Gegenstand der vorliegenden Erfindung sind Ammoniumsalze der Formel (I) 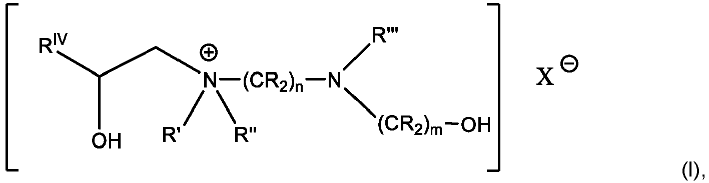 mit R = -H, -CH₃, -CH₂CH₃; n = 1, 2, 3, 4, 5; m = 2, 3; R', R", R‴ = -CH₃, -CH₂CH₃; R^{IV} = -H, -CH₃, -CH₂CH₃ und X = einwertiges Gegenion, Verfahren zu ihrer Herstellung und ihre Verwendung.

## Beschreibung

Die vorliegende Erfindung betrifft neue Ammoniumsalze, die als Katalysatoren, insbesondere als Katalysatoren für Umsetzungen mit Isocyanatgruppen-haltigen Verbindungen eingesetzt werden können. Die vorliegende Erfindung betrifft weiterhin ein Verfahren zu ihrer Herstellung und ihre Verwendung.

Reaktionen von Isocyanatgruppen-haltigen Verbindungen sind von großem Interesse für die technische Chemie. Insbesondere die Trimerisierung von Isocyanaten zu Isocyanuraten und die Umsetzung von Isocyanaten und Hydroxygruppen-haltigen Verbindungen zu Urethangruppen-haltigen Verbindungen sind von großer technischer Bedeutung, da die entsprechenden Produkte Verwendung in vielen Anwendungsfeldern finden.

Polyisocyanurate sind wertvolle Einsatzstoffe für die Herstellung hochwertiger Beschichtungen mit guten mechanischen Eigenschaften und guter Licht- und Wetterbeständigkeit. Polyisocyanurate auf Basis von Isophorondiisocyanat (IPDI) finden zudem Einsatz als Rohstoff für PUR-basierende Elastomeranwendungen.

Grundsätzlich werden Polyisocyanurate durch katalytische Trimerisierung geeigneter Isocyanate erhalten. Trimerisierbare Isocyanate sind z.B. aromatische, cycloaliphatische und aliphatische di- und höherwertige Polyisocyanate. Als Katalysatoren kommen z.B. tertiäre Amine (DE 24 52 532 A1), Komplexe von basischen Alkalimetallverbindungen und acyclischen organischen Verbindungen (EP 0 056 159 A1), Aminosilylgruppen-haltige Verbindungen (US 4,697,014) und quartäre Ammoniumsalze (US 4,503,226) in Betracht.

Besonders gut geeignet für die Trimerisierung von Isocyanaten zu Isocyanuraten sind weiterhin quartäre Hydroxyalkylammoniumsalze.

DE 26 31 733 A1 offenbart zum Beispiel die Katalyse von Trimerisierungen und Urethansynthesen mit quartären Hydroxyalkylammoniumsalzen, die eine beta-Hydroxyalkylgruppe aufweisen.

Auch DE 29 16 201 A1 lehrt, dass quartäre N-(Hydroxyalkyl)ammoniumsalze für die Trimerisierung von Isocyanaten zu Isocyanuraten eingesetzt werden können.

EP 1 454 933 A1 lehrt, dass für die Trimerisierung quartäre beta-hydroxylierte Ammoniumsalze verwendet werden können, deren quartäres Stickstoffatom drei Reste X trägt, die gemeinsam einen tricyclischen Ring bilden. Der tricyclische Ring wird über ein weiteres gemeinsames Stickstoffatom gebildet, das hydroxyalkyliert sein kann (und dann entsprechend ebenfalls quartär vorliegt).

DE 41 15 402 A1 offenbart schließlich in den Beispielen die Herstellung von N-(Hydroxyalkyl)-ammoniumsalzen, die für die Trimerisierung von Isocyanuraten eingesetzt werden können. Unter anderem wird immer jeweils ein Äquivalent N,N,N'-Trimethyl-N'-(β-hydroxyethyl)-ethylendiamin mit zwei Äquivalenten Monocarbonsäure umgesetzt und dann ein Monoepoxid im Überschuss zugegeben, so dass das resultierende Ammoniumsalz zwei quartäre Stickstoffatome aufweist.

Die im Stand der Technik offenbarten Ammoniumsalze lösen zwar bereits viele der Probleme, des Standes der Technik, wie z.B., dass die mit ihnen herstellbaren Isocyanurate lagerstabil, farbarm und geruchsarm sind. Nachteilig ist jedoch, dass die bekannten Katalysatoren noch nicht ausreichend aktiv sind.

Es ist somit die Aufgabe der vorliegenden Erfindung, Katalysatoren bereitzustellen, mit denen wie mit den bekannten Ammoniumsalzen lagerstabile, farbarme und geruchsarme Isocyanurate hergestellt werden können, die darüber hinaus jedoch den Vorteil aufweisen, aktiver zu sein.

Die sich vorliegend stellende Aufgabe wird gelöst durch das erfindungsgemäße Ammoniumsalz mit der nachfolgenden Formel (I) 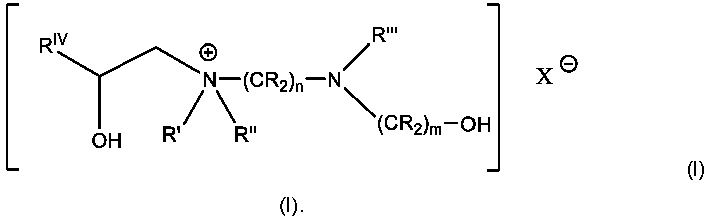 mit
R = -H, -CH₃, -CH₂CH₃
n = 1, 2, 3, 4, 5,
m = 2, 3,
R', R", R‴ = -CH₃, -CH₂CH₃
R^{IV} = H, -CH₃, -CH₂CH₃
und X = einwertiges Gegenion.

Dabei können die Reste R unabhängig voneinander aus der Gruppe bestehend aus -H, -CH₃, -CH₂CH₃ ausgewählt werden.

Die erfindungsgemäßen Ammoniumsalze sind Ammoniumsalze, die durch Umsetzung eines ditertiären Amins, dessen eine tertiäre Aminogruppe mit einer Hydroxyalkylgruppe substituiert ist, mit einem Alkylenoxid und einer Säure HX herstellbar sind. Liegt Alkylenoxid und Säure nicht im molaren Überschuss, bezogen auf die Gesamtmenge an ditertiärem Amin, vor, reagiert überraschenderweise nur die Aminogruppe ohne die Hydroxyalkylgruppe -(CR₂)ₘ-OH mit dem Alkylenoxid. Entsprechende Salze mit einer Ammonium- und einer tertiären Aminogruppe im Kation sind überraschenderweise aktiver als Diammoniumsalze.

Bevorzugte Katalysatoreigenschaften entstehen, wenn sich die Ammoniumsalze von Säuren HX ableiten, die ausgewählt sind aus der Gruppe der Carbonsäuren. Weiter bevorzugt werden die Carbonsäuren ausgewählt aus der Gruppe der aliphatischen und heterocyclischen Carbonsäuren. Noch weiter bevorzugt ist die Carbonsäure eine aliphatische Carbonsäure. Noch weiter bevorzugt ist die Carbonsäure eine Alkansäure oder Alkensäure. Besonders bevorzugte Carbonsäuren können ausgewählt werden aus der Gruppe der Alkansäuren mit der Formel R^{V}COOH mit R^{V} = CᵣH₂ᵣ₊₁ mit r = 1 - 9. Entsprechende Ammoniumsalze weisen die Formel (II) auf:

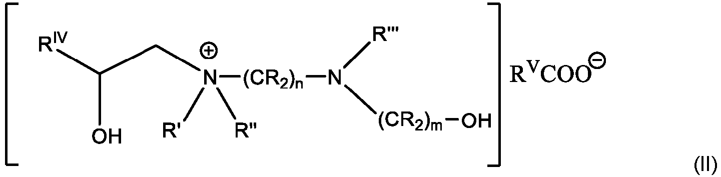

In den Formeln (I) bzw. (II) sind die Reste R unabhängig voneinander ausgewählt aus der Gruppe bestehend aus -H, -CH₃ und -CH₂CH₃. Bevorzugt ist R unabhängig voneinander ausgewählt aus der Gruppe bestehend aus -H und -CH₃. Noch weiter bevorzugt ist R unabhängig voneinander ausgewählt aus der Gruppe bestehend aus -H und -CH₃, mit der Maßgabe, dass jedes Kohlenstoffatom maximal einen Rest R trägt, der ein -CH₃-Rest ist. Noch weiter bevorzugt sind alle R = -H.

In den Formeln (I) bzw. (II) beträgt der Index n eine Zahl ausgewählt aus 1, 2, 3, 4 und 5. Bevorzugt ist n eine Zahl ausgewählt aus 2, 3 und 4. Noch weiter bevorzugt ist n = 2 oder 3. Ganz besonders bevorzugt ist n = 2.

In den Formeln (I) bzw. (II) beträgt der Index m eine Zahl ausgewählt aus 2 und 3. Noch weiter bevorzugt ist m = 2.

Die Reste R', R" und R‴ können unabhängig voneinander ausgewählt werden aus der Gruppe bestehend aus -CH₃ und -CH₂CH₃. Bevorzugt ist jeder Rest R', R" und R‴ = -CH₃.

Der Rest R^{IV} in den Formeln (I) und (II), der sich von dem zur Herstellung des Ammoniumsalzes eingesetzten Alkylenoxid ableitet, ist ausgewählt aus der Gruppe bestehend aus -H, -CH₃ und -CH₂CH₃. Entsprechend leitet sich der Rest R^{iV} von einem Alkylenoxid ausgewählt aus Ethylenoxid, Propylenoxid und α-Butylenoxid ab. Bevorzugt ist der Rest R^{IV} ausgewählt aus der Gruppe bestehend aus -CH₃ und -CH₂CH₃. Aufgrund der Kombination von vergleichsweise geringer Toxizität und einfachem Handling (vor allem aufgrund des relativ hohen Siedepunkts) des zur entsprechenden Synthese erforderlichen α-Butylenoxids ist ganz besonders bevorzugt R^{IV} - CH₂CH₃.

Gegenstand der vorliegenden Erfindung ist weiterhin ein Verfahren zu Herstellung der erfindungsgemäßen Ammoniumsalze der Formel (II), bei dem ein ditertiäres Amin, dessen eine tertiäre Aminogruppe mit einer Hydroxyalkylgruppe substituiert ist, mit einem Alkylenoxid und einer Säure HX, bevorzugt einer Alkansäure, insbesondere einer Alkansäure mit der Formel R^{V}COOH mit R^{V} = CᵣH₂ᵣ₊₁ mit r = 1 - 9, umgesetzt wird.

Bevorzugt wird dabei das Alkylenoxid in einem Stoffmengenverhältnis von 0,9 - 1,1, weiter bevorzugt 0,95 - 1,05, noch weiter bevorzugt 0,98 - 1,02, und die Säure in einem Stoffmengenverhältnis von 0,9 - 1,1, weiter bevorzugt 0,95 - 1,05, noch weiter bevorzugt 0,98 - 1,02, jeweils bezogen auf die Stoffmenge des ditertiären Amins, eingesetzt.

Das ditertiäre Amin weist bevorzugt die nachfolgend dargestellte Formel (III) 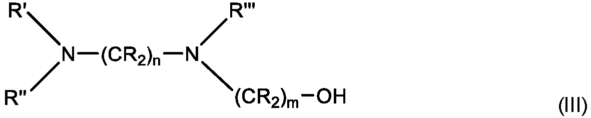 mit
R = -H, -CH₃, -CH₂CH₃
n = 1, 2, 3, 4, 5,
m = 2, 3, und
R', R", R‴ = -CH₃, -CH₂CH₃ auf.

Der Rest R wird unabhängig voneinander ausgewählt aus der Gruppe bestehend aus -H, -CH₃ und -CH₂CH₃. Bevorzugt ist R unabhängig voneinander ausgewählt aus der Gruppe bestehend aus -H und -CH₃. Noch weiter bevorzugt ist R unabhängig voneinander ausgewählt aus der Gruppe bestehend aus -H und -CH₃, mit der Maßgabe, dass jedes Kohlenstoffatom maximal einen Rest R trägt, der ein -CH₃-Rest ist. Noch weiter bevorzugt sind alle R = -H.

Der Index n ist eine Zahl ausgewählt aus 1, 2, 3, 4 und 5. Bevorzugt ist n eine Zahl ausgewählt aus 2, 3 und 4. Noch weiter bevorzugt ist n = 2 oder 3. Ganz besonders bevorzugt ist n = 2.

Der Index m ist eine Zahl ausgewählt aus 2 und 3. Noch weiter bevorzugt ist m = 2.

Die Reste R', R" und R‴ können unabhängig voneinander ausgewählt werden aus der Gruppe bestehend aus -CH₃ und -CH₂CH₃. Bevorzugt ist jeder Rest R', R" und R‴ = -CH₃.

Bevorzugt werden Säuren HX eingesetzt, die ausgewählt sind aus der Gruppe der Carbonsäuren. Weiter bevorzugt werden die Carbonsäuren ausgewählt aus der Gruppe der aliphatischen und heterocyclischen Carbonsäuren. Noch weiter bevorzugt ist die Carbonsäure eine aliphatische Carbonsäure. Noch weiter bevorzugt ist die Carbonsäure eine Alkansäure oder Alkensäure. Besonders bevorzugte Carbonsäuren können ausgewählt werden aus der Gruppe der Alkansäuren. Besonders bevorzugte Alkansäuren können ausgewählt werden aus der Gruppe der Alkansäuren mit der Formel R^{V}COOH mit R^{V} = CᵣH₂ᵣ₊₁ mit r = 1 - 9.

Noch weiter bevorzugt ist das Alkylenoxid ausgewählt aus Ethylenoxid, Propylenoxid und α-Butylenoxid. Entsprechende Produkte fallen unter die Formel (III). Weiter bevorzugt ist das Alkylenoxid ausgewählt aus Propylenoxid und α-Butylenoxid. Aufgrund der Kombination von vergleichsweise geringer Toxizität und einfachem Handling (vor allem aufgrund des relativ hohen Siedepunkts) ist das Alkylenoxid ganz besonders bevorzugt α-Butylenoxid.

Das erfindungsgemäße Verfahren wird bevorzugt so durchgeführt, dass zunächst das ditertiäre Amin mit der Säure vermischt wird und nachfolgend das Alkylenoxid hinzugegeben wird.

Das erfindungsgemäße Verfahren kann prinzipiell in An- oder Abwesenheit eines Lösemittels durchgeführt werden. Bevorzugt wird es jedoch in Abwesenheit eines Lösemittels durchgeführt. Bevorzugte Reaktionstemperaturen liegen zwischen RT und 120°C, weiter bevorzugte zwischen 40 und 80°C. Die Reaktion kann z.B. gaschromatographisch auf Vollständigkeit geprüft werden. Sobald kein Alkylenoxid mehr nachweisbar ist, wird die Reaktion abgebrochen.

Gegenstand der vorliegenden Erfindung ist weiterhin die Verwendung der erfindungsgemäßen Ammoniumsalze als Katalysatoren. Bevorzugt ist weiterhin die Verwendung der erfindungsgemäßen Ammoniumsalze als Katalysatoren für die Trimerisierung von Isocyanaten zu Isocyanuraten oder für die Umsetzung von Isocyanaten und Hydroxygruppen-haltigen Verbindungen zu Urethangruppen-haltigen Verbindungen. Ganz besonders geeignet sind die erfindungsgemäßen Ammoniumsalze als Katalysatoren für die Trimerisierung von Isocyanaten zu Isocyanuraten.

### Beispiele

### Erfindungsgemäßes Beispiel A:

146 Teile N,N,N'-Trimethyl-N'-(hydroxyethyl)-ethylendiamin (1 Mol Diamin) werden mit 116 Teilen (1 Mol) Hexansäure gemischt und dann bei 40 °C portionsweise und unter Rückfluss und Rühren mit 72 Teilen (1 Mol) 1,2-Butylenoxid versetzt. Nach vollständiger Zugabe wird noch 96 h bei 40 °C gerührt, wonach im GC kein 1,2-Butylenoxid mehr nachweisbar ist. Laut NMR hat lediglich der Dimethylamin Stickstoff reagiert.

### Nicht erfindungsgemäßes Beispiel B (gemäß DE4115102)

146 Teile N,N,N'-Trimethyl-N'-(hydroxyethyl)-ethylendiamin (1 Mol Diamin) werden mit 232 Teilen (2 Mol) Hexansäure gemischt und dann bei 40 °C portionsweise und unter Rückfluss und Rühren mit 144 Teilen (2 Mol) 1,2-Butylenoxid versetzt. Nach vollständiger Zugabe wird noch 96 h bei 40 °C gerührt, wonach im GC kein 1,2-Butylenoxid mehr nachweisbar ist.

### Trimerisierung

### Allgemeine Vorschrift:

1500 g Isophorondiisocyanat (IPDI) werden auf 70 °C aufgeheizt und danach erst mit 3 g Katalysator versetzt und dann gerührt. Dabei wird die Zeitdauer der Exothermie, das Maximum der Temperaturkurve und die endgültige NCO-Zahl bestimmt.

| | Zeitdauer der Exothermie [min] | Maximum der Temperaturkurve [°C] | NCO-Zahl nach Reaktion (Start 37,8) |
|---|---|---|---|
| Beispiel A | 2:30 | 156 | 24,5 |
| Beispiel B | 2:43 | 137 | 28,6 |

Die entstehenden Teiltrimerisate sind sowohl stabil, farblos als auch geruchsarm. Der erfindungsgemäße Katalysator mit einer Ammonium- und einer tertiären Aminogruppe im kationischen Anteil ist deutlich aktiver als der Katalysator des Vergleichsbeispiels mit zwei Ammoniumgruppen im kationischen Anteil.

## Patentansprüche

1. Ammoniumsalz der Formel (I) 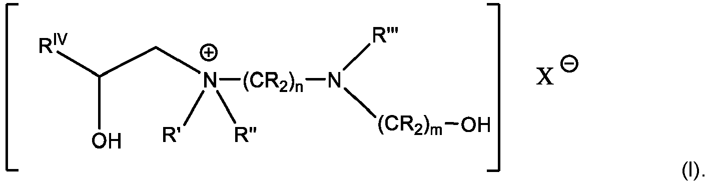 mit
R = -H, -CH₃, -CH₂CH₃
n = 1, 2, 3, 4, 5
m = 2, 3,
R', R", R‴ = -CH₃, -CH₂CH₃
R^{IV} = -H, -CH₃, -CH₂CH₃
und X = einwertiges Gegenion.

2. Ammoniumsalz nach Anspruch 1,
**dadurch gekennzeichnet, dass**
es die Formel (II) 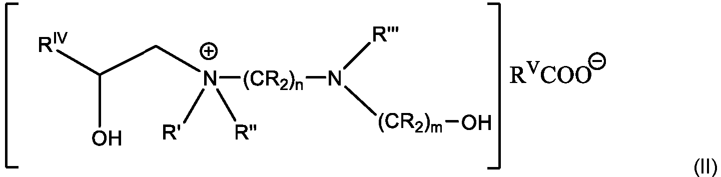
mit R^{V} = CᵣH₂ᵣ₊₁ und r = 1 - 9 aufweist.

3. Ammoniumsalz nach einem der vorhergehenden Ansprüche,
**dadurch gekennzeichnet, dass**
R = -H ist.

4. Ammoniumsalz nach einem der vorhergehenden Ansprüche,
**dadurch gekennzeichnet, dass**
n = 2 oder 3.

5. Ammoniumsalz nach einem der vorhergehenden Ansprüche,
**dadurch gekennzeichnet, dass**
m = 2.

6. Ammoniumsalz nach einem der vorhergehenden Ansprüche,
**dadurch gekennzeichnet, dass**
R' = R" = R‴ = -CH₃.

7. Ammoniumsalz nach einem der vorhergehenden Ansprüche,
**dadurch gekennzeichnet, dass**
R^{IV} = -CH₂CH₃.

8. Verfahren zur Herstellung eines Ammoniumsalzes nach einem der vorhergehenden Ansprüche,
**dadurch gekennzeichnet, dass**
ein ditertiäres Amin, dessen eine tertiäre Aminogruppe mit einer Hydroxyalkylgruppe substituiert ist, mit einem Alkylenoxid und einer Säure HX umgesetzt wird.

9. Verfahren nach Anspruch 8,
**dadurch gekennzeichnet, dass**
das Alkylenoxid in einem Stoffmengenverhältnis von 0,9 - 1,1 und die Säure in einem Stoffmengenverhältnis von 0,9 - 1,1, jeweils bezogen auf die Stoffmenge des ditertiären Amins, eingesetzt wird.

10. Verfahren nach einem der Ansprüche 8 oder 9,
**dadurch gekennzeichnet, dass**
dass das ditertiäre Amin die Formel 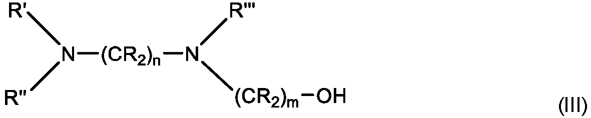 mit
R = -H, -CH₃, -CH₂CH₃
n = 1, 2, 3, 4, 5,
m = 2, 3, und
R', R", R" = -CH₃, -CH₂CH₃ aufweist.

11. Verfahren nach Anspruch 10,
**dadurch gekennzeichnet, dass**
die Säure HX eine Alkansäure ausgewählt aus der Gruppe der Alkansäuren mit der Formel R^{V}COOH mit R^{V} = CᵣH₂ᵣ₊₁ mit r = 1 - 9 ist.

12. Verfahren nach Anspruch 11,
**dadurch gekennzeichnet, dass**
das Alkylenoxid ausgewählt aus Ethylenoxid, Propylenoxid und α-Butylenoxid ist.

13. Verwendung eines Ammoniumsalzes nach einem der Ansprüche 1 - 7 als Katalysator, insbesondere für die Trimerisierung von Isocyanaten zu Isocyanuraten oder für die Umsetzung von Isocyanaten und Hydroxygruppen-haltigen Verbindungen zu Urethangruppen-haltigen Verbindungen.
